# EUROPEAN PATENT APPLICATION

(11) **EP 2 034 333 A2**
(43) Date of publication of application: **11.03.2009**
(21) Application number: 08015530.2
(22) Date of filing: 03.09.2008
(51) Int. Cl.: G01S 15/89

(54) **Ultrasound system and method of forming a 3D colour flow ultrasound image**

(30) Priority: 04.09.2007 KR 20070089243
(71) Applicant: MEDISON CO., LTD., Kangwon-do 250-870 (KR)
(72) Inventor: Hyun, Dong Gyu, Seoul 135-280 (KR)
(74) Representative: Lorenz, Markus

(57) **Abstract**

The present invention relates to an ultrasound system, which includes: a transmit/receive unit for transmitting ultrasound signals toward a target object having reflectors along scan lines and receiving ultrasound echo signals reflected from the target object to form receive signals based on the ultrasound echo signals; a signal processing unit for forming image signals based on the receive signals, the image signals being indicative of locations and velocities of the reflectors in the target object; and an image processing unit for forming a 3-dimensional image 3-dimensionally indicating the velocities of the reflectors based on the image signals.

## Description

The present application claims priority from Korean Patent Application No. 10-2007-0089243 filed on September 4, 2007, the entire subject matter of which is incorporated herein by reference.

### BACKGROUND OF THE INVENTION

### [Technical Field]

The present invention generally relates to ultrasound systems, and more particularly to an ultrasound system and a method of forming an ultrasound image.

### [Background Art]

An ultrasound system has become an important and popular diagnostic tool due to its non-invasive and non-destructive nature. Modern high-performance ultrasound imaging diagnostic systems and techniques are commonly used to produce two- or three-dimensional images of internal features of patients.

The ultrasound system may provide a color flow image, which shows blood flow information. The blood flow information may include information about a plurality of blood flow velocities at the target object. The velocities may be computed at the target object by using the Doppler effect. The color flow image is an image indicating the velocities with predetermined colors corresponding to the respective velocities. The color flow image not only provides real-time blood flow visualization but can also accurately delineate a wide range of blood flow conditions, ranging from high velocities in large vessels to minute trickles coursing through small vessels.

The conventional ultrasound image may merely provide the color flow image showing velocity information at the target object without indicating velocity changes of the reflectors in the target object. Thus, it is difficult for a user to intuitively recognize the velocity change of the reflectors in the target object.

### BRIEF DESCRIPTION OF THE DRAWINGS

FIG. 1 is a block diagram showing an illustrative embodiment of an ultrasound system.

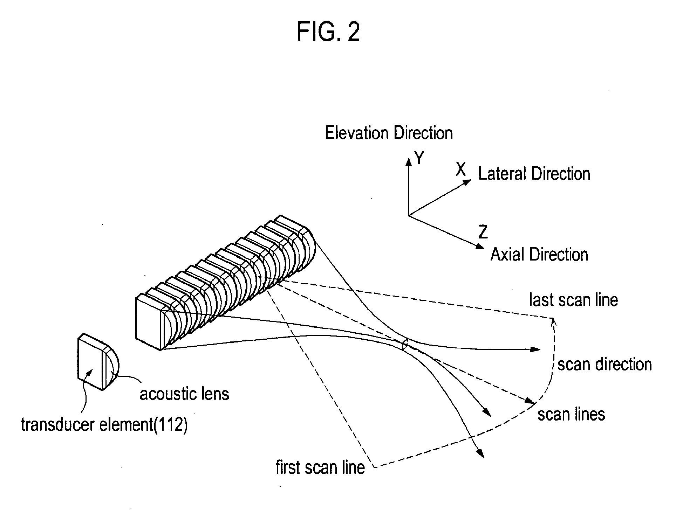

FIG. 2 is a schematic diagram illustrating an exemplary configuration of transducer elements and acoustic lens as well as further showing scan lines and a coordinates system.

FIGS. 3 to 6 are exemplary diagrams showing illustrative embodiments of 3-dimensional images showing velocity changes in a target object.

FIG. 7 is a schematic diagram showing an example of a display where a reference plane is set on a 3-dimensional image.

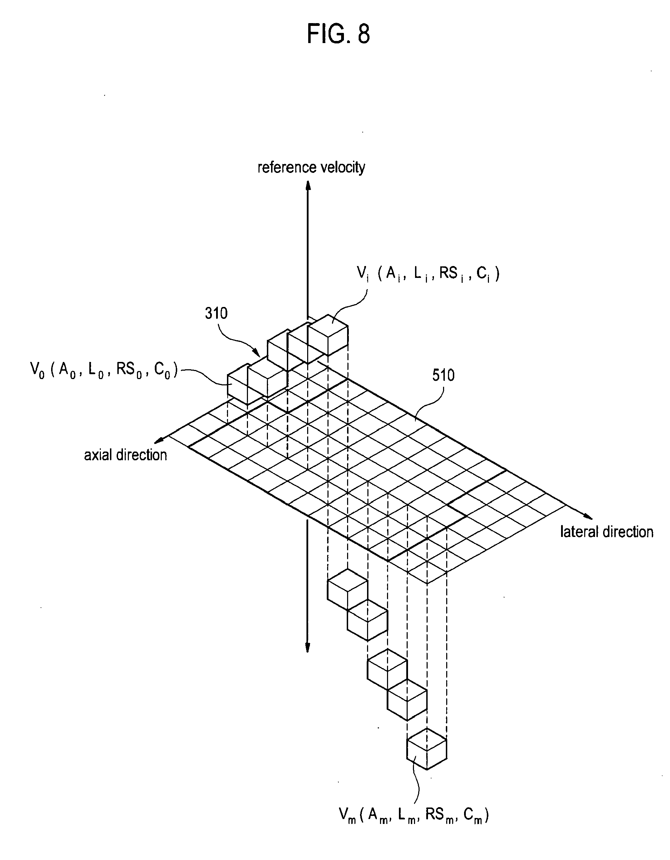

FIG. 8 is a schematic diagram showing an example of a display where a 2-dimensional image is displayed together with a 3-dimensional image.

### DETAILED DESCRIPTION OF THE INVENTION

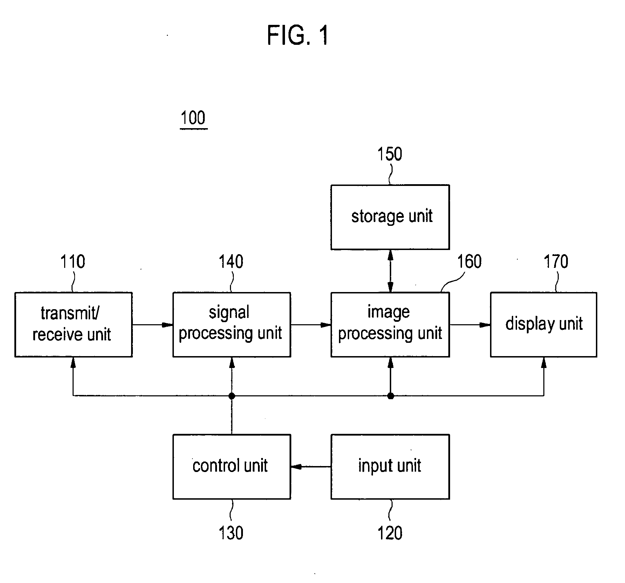

FIG. 1 is a block diagram showing an illustrative embodiment of an ultrasound system. As shown in FIG. 1, the ultrasound system 100 may include a transmit/receive unit 110, an input unit 120, a control unit 130, a signal processing unit 140, a storage unit 150, an image processing unit 160 and display unit 170.

The transmit/receive unit 110 may include a probe (not shown) containing a plurality of transducer elements 112 for reciprocally converting the ultrasound signals and the electric signals. The probe may transmit ultrasound signals along a plurality of scan lines set in a target object and receive ultrasound echo signals reflected from the target object under the control of the control unit 130. The transmit/receive unit 110 may further include a transmitter and a receiver. The transmitter may be operable to form a transmit pattern of transmit pulses, which are applied to transducer elements, such that the ultrasound signals generated from the transducer elements are focused on focal points on the scan lines. The receiver may be configured to perform receive focusing, i.e., apply delays to the receive signals in consideration of distances between the transducer elements and the focal points.

The input unit 120 may allow the user to input instructions upon setup information. The setup information may include information about an image mode of the ultrasound system and information about a location and a size of a region of interest. The setup information may further include information for setting a reference plane. The input unit 120 may be any user interface unit such as a mouse, a keyboard, a track ball or the like.

The control unit 130 may control the transmit/receive unit 110 of the ultrasound system. For example, as for an instruction about the image mode of the ultrasound system, the control unit 130 may be operable to control the transmit/receive unit 110 so that the transmit/receive unit 110 may obtain receive signals corresponding to the inputted image mode based on the ultrasound echo signals.

If the image mode is a B-mode, then the receive signals may be B-mode receive signals. Also, if the image mode is a Doppler mode, then the receive signals may be Doppler signals. The target object may include moving objects such as a blood flow or a heart. Further, if the setup information upon the region of interest and a Doppler mode are sequentially inputted while the B-mode image is displayed, then the transmit/receive unit 110 may obtain Doppler signals based on the ultrasound echo signals. In one embodiment, the region of interest may include a color box. The control unit 130 may be further operable to control the signal processing unit 140, the image processing unit 160 and the display unit 170.

The signal processing unit 140 may perform one of signal processing upon the receive signals. For example, if the receive signals are the B-mode receive signals, then the signal processing unit 140 may form 2-dimensional B-mode image signals. Also, the signal processing unit 140 may perform signal processing upon the Doppler signals to thereby form 3-dimensional color flow image signals. The 3-dimensional color flow image signals may be indicative of a plurality of reflector velocities at the target object. In one embodiment, the 3-dimensional color flow image signals may be further indicative of location information on the reflectors within the region of interest in axial and lateral directions (2-dimensional location).

In another embodiment, the B-mode image signals may be indicative of the 2-dimensional location of the reflectors and intensities of the ultrasound echo signals. The color flow image signals may be indicative of the 2-dimensional location of the reflectors and the reflector velocities within the region of interest.

The storage unit 150 may store a first mapping table between colors and velocities, as well as a second mapping table between colors and intensities. The storage unit 150 may further store a third mapping table between velocities and intensities. The storage unit 150 may include any one of non-volatile storage devices such as a flash memory, a hard disk, a CD ROM and the like.

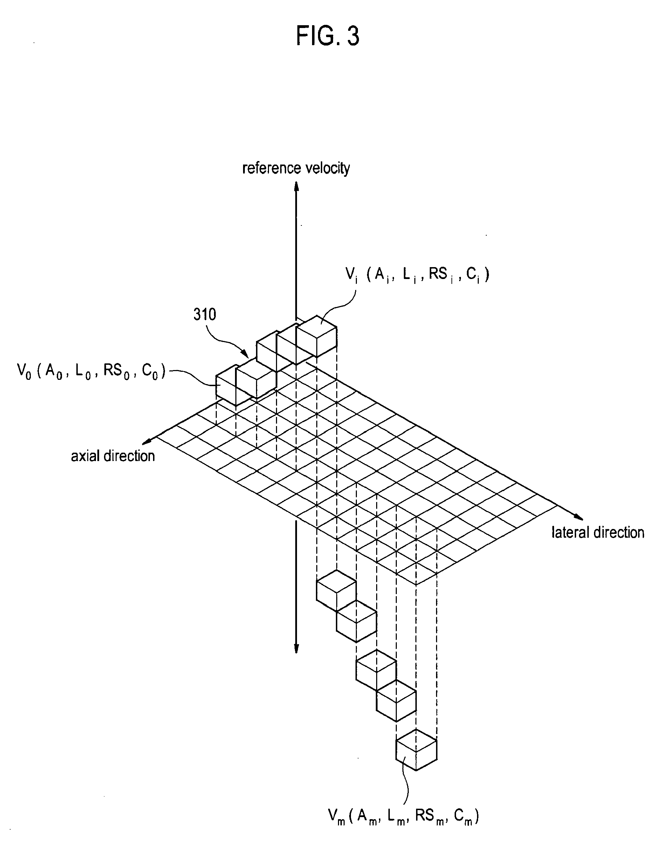

The image processing unit 160 may be operable to form a 2-dimensional B-mode image based on the 2-dimensional B-mode image signals. Further, the image processing unit 160 may form a plurality of voxels V₀ to Vₘ based on the color flow image signals, as shown in FIG. 3. In order to form the voxels, the image processing unit 160 may be operable to obtain a plurality of reflector velocities based on the color flow image signals, and then set a reference velocity from the plurality of reflector velocities. In such a case, the reference velocity may be an average velocity, a minimum velocity or a maximum velocity of the plurality of reflector velocities. The voxels may be formed to indicate the reference velocities on a 3-dimensional space defined by the axial and lateral directions (A, L) and the reference velocity direction RS. Each of the voxels may be indicated with 3-dimensional location (A, L, RS). The voxels may be matched with the respective pixels at a slice within the region of interest set on the 2-dimensional B-mode image. Each of the voxels may have an arbitrary shape such as a cube. The image processing unit may be operable to refer to the first mapping table stored in the storage unit 150 to thereby indicate each of the voxels by a color corresponding to the reference velocity. In one embodiment, each of the voxels may be represented by, for example, Vo(Ao, L₀, RS₀, Co). A₀ may represent a location in an axial direction, L₀ may represent a location in a lateral direction, RS₀ may represent a reference velocity and the Co may represent a color of the corresponding voxel. The image processing unit 160 may form a 3-dimensional color flow image 310 with the plurality of the voxels.

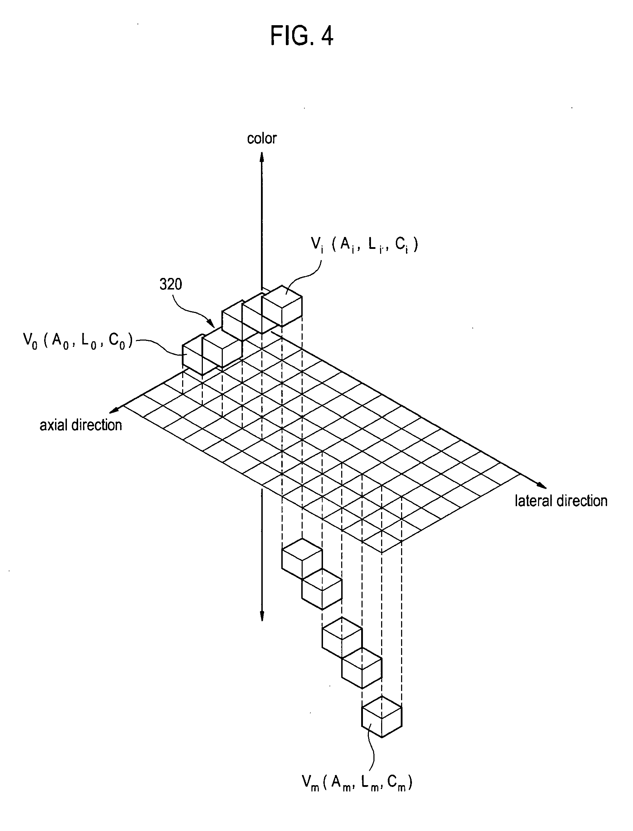

Also, the image processing unit 160 may be operable to form 3-dimensional cimage 320 on a 3-dimensional space defined by the axial and lateral directions (A, L) and a color direction C, as shown in FIG. 4. In such a case, the voxels may be formed to show colors corresponding to the reference velocities and indicated by 3-dimensional location (A, L, C). The image processing unit 160 may be operable to refer to the first mapping table stored in the storage unit 150 to thereby indicate each of the voxels with a color corresponding to the reference velocity. In such a case, each of the voxels may be represented by, for example, Vo(Ao, L₀, Co). Ao may represent a location in an axial direction, L₀ may represent a location in a lateral direction and Co may represent a color of the corresponding voxel.

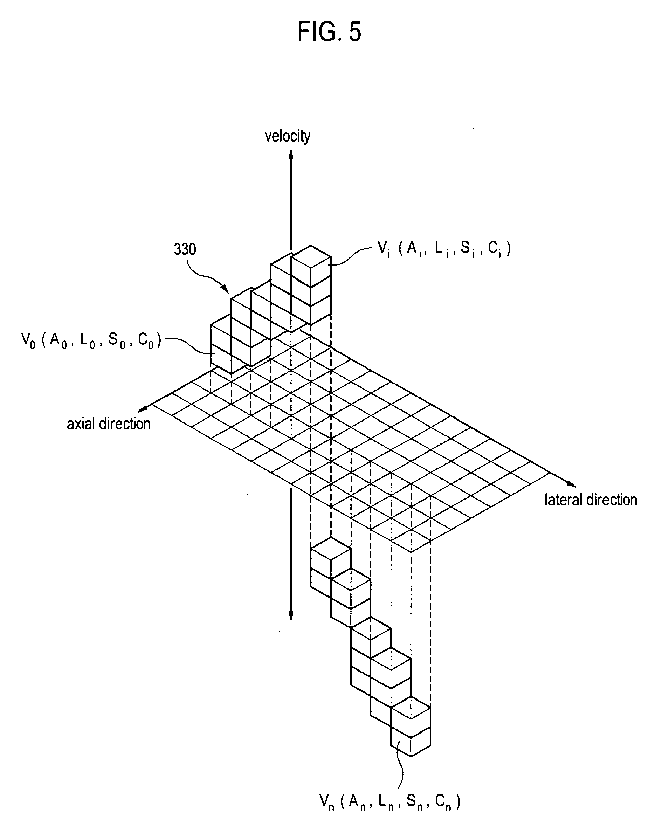

In accordance with another embodiment, the image processing unit 160 may be operable to form the 3-dimensional color flow image 330 on a 3-dimensional space defined by the axial and lateral directions (A, L) and a current velocity direction S, as shown in FIG. 5. In such a case, the voxels may be formed to show colors corresponding to the current velocities of the reflectors and indicated with 3-dimensional location (A, L, S). The image processing unit 160 may be operable to refer to the first mapping table stored in the storage unit 150 to thereby indicate each of the voxels by a color corresponding to the current velocity S. In such a case, each of the voxel may be represented by, for example, Vo(Ao, L₀, S₀, Co). Ao may represent a location in an axial direction, L₀ may represent a location in a lateral direction, S₀ may represent a current velocity and Co may represent a color of the corresponding voxel.

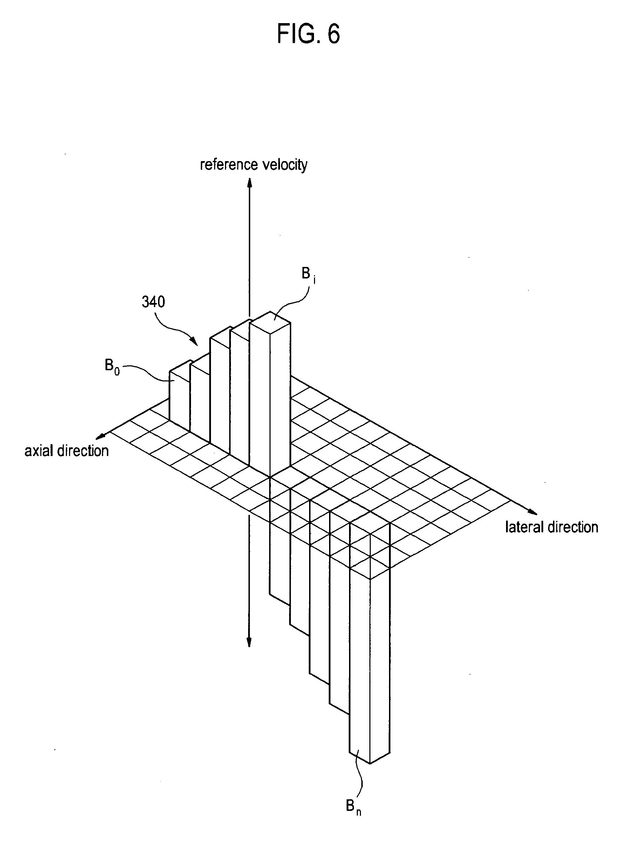

In another embodiment, the image processing unit 160 may form the 3-dimensional color flow image 340 with bar graphs Bo to Bₙ on a 3-dimensional space defined by the axial and lateral directions (A, L) and a reference velocity RS, as shown in FIG. 6. In such a case, the bar graphs may be formed to indicate the reference velocities. Each of the graphs may be indicated with a color C corresponding to the reference velocity at each of the voxels. In such a case, the height of each of the bar graphs may represent a reference velocity.

In accordance with still another embodiment, the image processing unit 160 may be further operable to form a 3-dimensional color flow image on a 3-dimensional space defined by the axial and lateral directions (A, L) and intensity based on the first receive signals. The image processing unit 160 may be operable to set velocities corresponding to the intensities within the region of interest based on the B-mode image signals. In such a case, the image processing unit 160 may be operable to retrieve the third mapping table stored in the storage unit 150 to set the velocities corresponding to the respective intensities. The image processing unit 160 may be operable to form the 3-dimensional image constructed with a plurality of voxels, wherein each of the voxels is indicative of the intensity. Each of the voxels may be indicated with a color corresponding to the intensity of each of the voxels.

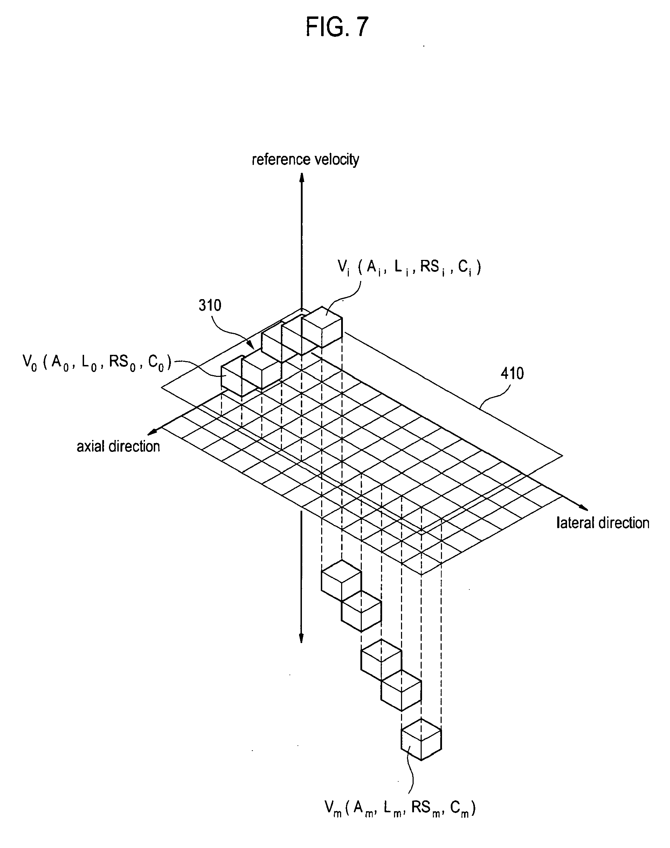

The image processing unit 160 may be operable to perform a variety of image processing upon the 3-dimensional image. The image process may be operable to set a reference plane 410 in the 3-dimensional color flow image 310 based on the reference plane setting information inputted through the input unit 120 from the user, and then form a reference plane image corresponding to the reference plane 410, as illustrated in FIG. 7. In accordance with one embodiment, although an example of setting the reference plane on the 3-dimensional color flow image 310 illustrated in FIG. 3 is described, it is certainly not limited thereto. The reference plane may be set on any 3-dimensional color flow image described in the above embodiments and the reference plane image corresponding to the reference plane may be formed.

Further, the image processing unit 160 may be operable to perform perspective projection or orthographic projection upon the 3-dimensional image to form a 3-dimensional projection image in accordance with one embodiment of the present invention.

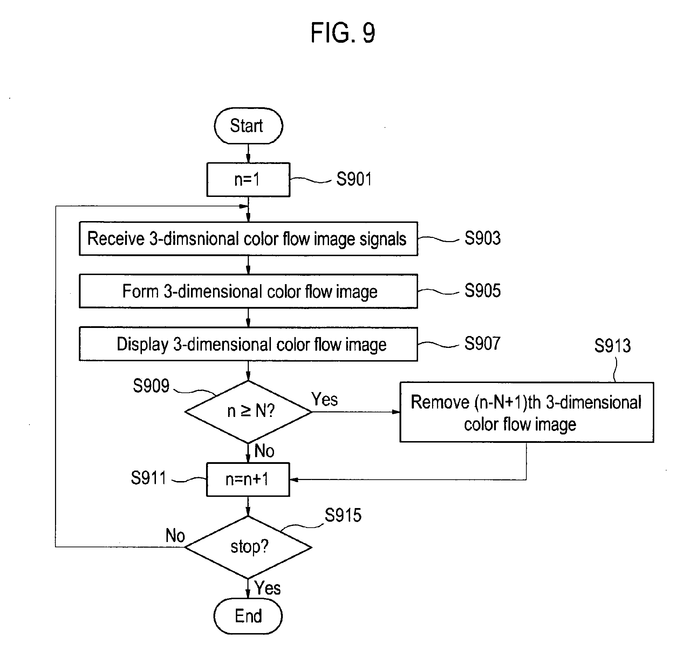

The image processing unit 160 may be operable to perform image processing to display a predetermined number of 3-dimensional color flow images for a preset time through the display unit 170 in real time. For example, a display of the 3-dimensional color flow image will be described by referring to FIG. 9.

If n = 1 at step S901, then the image processing unit 160 may receive color flow image signals from the signal processing unit 140 at step S903, In such a case, n represents the number of displaying 3-dimensional color flow images. The image processing unit 160 may form a 1^{st} 3-dimensional color flow image IM₁ based on the 3-dimensional color flow image signals at step S905. The formed 3-dimensional image IM₁ is displayed through the display unit 170 at step S907.

Subsequently, the image processing unit 160 may check whether the number of the displayed 3-dimensional color flow images is equal to or greater than a predetermined number (e.g., 5) at step S909. If it is determined that the number of the displayed 3-dimensional color flow images is less than the predetermined number, then n = n+1 (S911). In such a case, the image processing unit 160 may check whether an instruction for stopping displaying the 3-dimensional color flow images is inputted at step S915. If not, then the step goes to the step S903. On the contrary, if it is determined that the number of the displayed 3-dimensional color flow images is equal to or greater than the predetermined number, then the image processing unit 160 may remove (n-N+1)^{th} 3-dimensional color flow image from the displayed 3-dimensional color flow images at step S913 and then the process goes to the step S915.

In one embodiment, when the plurality of 3-dimensional color flow images are displayed at the same time, the previously formed 3-dimensional color flow images may be displayed with relatively lower brightness than the currently formed 3-dimensional color flow images by applying a predetermined weight. For example, when the 3-dimensional color flow images IM₁ and IM₂ are displayed, the image processing unit 160 may apply a first weight (e.g., 0.8) to the 3-dimensional color flow image IM₁. Also, when the 3-dimensional color flow images IM₁, IM₂ and IM₃ are displayed at the same time, the image processing unit 160 may apply a second weight (e.g., 0.6) to the 3-dimensional color flow image IM1 and the second weight to the 3-dimensional color flow image IM₂. The above process may be repeatedly carried out until the instruction for stopping displaying the 3-dimensional color flow images. In one embodiment, although it is described that five 3-dimensional color flow images are displayed on the display unit 170 at the same time, the number of displaying the 3-dimensional images is certainly not limited thereto. It should be understood that the number of the 3-dimensional images to be simultaneously displayed and the weight may be changed according to the necessity by those skilled in the art.

The display unit 170 may display the 2-dimensional image, the 3-dimensional color flow image and the reference plane image. The display unit 170 may be operable to display only the 3-dimensional color flow image. Also, the display unit 170 may display the 2-dimensional image together with the 2-dimensional image. Further, the display unit 170 may be operable to display the 2-dimensional image, the 3-dimensional color image and the reference plane image at the same time.

As mentioned above, since the 3-dimensional color flow image showing the velocity changes at the respective locations in the target object is provided, the user may intuitively recognize the velocity changes in the target object.

In accordance with one embodiment of the present invention, there is provided an ultrasound system, comprising a transmit/receive unit operable to transmit ultrasound signals toward a target object having reflectors along scan lines and receive ultrasound echo signals reflected from the target object to form receive signals based on the ultrasound echo signals; a signal processing unit operable to form image signals based on the receive signals, the image signals being indicative of locations and velocities of the reflectors in the target object; and an image processing unit operable to form a 3-dimensional image 3-dimensionally indicating the velocities of the reflectors based on the image signals.

In accordance with another embodiment of the present invention, there is provided a method of forming an ultrasound image, comprising: a) transmitting ultrasound signals along scan lines set in a target object having reflectors and receiving ultrasound echo signals reflected from the target object to form receive signals based on the ultrasound echo signals; b) forming image signals based on the receive signals, the image signals being indicative of locations and velocities of the reflectors in the target object; and c) forming a 3-dimensional image 3-dimensionally indicating reflector velocities based on the image signals.

Any reference in this specification to "one embodiment," "an embodiment," "example embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a transmit/receive unit for transmitting ultrasound signals toward a target object having reflectors along scan lines and receiving ultrasound echo signals reflected from the target object to form receive signals based on the ultrasound echo signals;
a signal processing unit for forming image signals based on the receive signals, the image signals being indicative of locations and velocities of the reflectors in the target object; and
an image processing unit for forming a 3-dimensional image 3-dimensionally indicating the velocities of the reflectors based on the image signals.

2. The ultrasound system of Claim 1, further comprising a storage unit configured to a mapping table providing information between colors corresponding to the respective velocities.

3. The ultrasound system of Claim 2, wherein the image processing unit is configured to:
obtain the plurality of reflector velocities based on the image signals;
set a reference velocity from the plurality of velocities at each of the reflectors;
form voxels to indicate 3-dimensional locations based on the image signals and the reference velocity and indicated by a color corresponding to the reference velocity of each reflector by referring to the mapping table; and
form a 3-dimensional image with the voxels.

4. The ultrasound system of Claim 2, wherein the image processing unit is configured to:
set a reference velocity from the plurality of reflector velocities and retrieve the mapping table to set a color corresponding to the reference velocity;
form voxels to indicate 3-dimensional location information based on the image signals and the color corresponding to the reference velocity and indicated by the color corresponding to the reference velocity; and
form a 3-dimensional image constructed with a plurality of voxels.

5. The ultrasound system of Claim 2, wherein the image processing unit is configured to form a 3-dimensional image constructed with a plurality of voxels, wherein each of the voxels is formed to indicate 3-dimensional location based on the location of the reflector and a current reflector velocity and indicated by the color corresponding to the current velocity.

6. The ultrasound system of Claim 2, wherein the image processing unit is configured to:
obtain the plurality of reflector velocities and set colors corresponding to the reflector velocities by referring to the mapping table; and
form a 3-dimensional image constructed with a plurality of voxels, wherein each of the voxels is formed to indicate 3-dimensional location based on the location of the reflector and the color corresponding to the reflector velocity and indicated by the color corresponding to the reference velocity.

7. The ultrasound system of Claim 2, wherein the image processing unit is configured to:
set a reference velocity from the plurality of reflector velocities and set a color corresponding to the reference velocity by referring to the mapping table; and
form a 3-dimensional image constructed with a plurality of bar graphs, wherein each of the graphs is formed to indicate 3-dimensional location based on the location and the reference velocity of the reflector and indicated by the color corresponding to the reference velocity.

8. A method of forming an ultrasound image, comprising:
a) transmitting ultrasound signals along scan lines set in a target object having reflectors and receiving ultrasound echo signals reflected from the target object to form receive signals based on the ultrasound echo signals;
b) forming image signals based on the receive signals, the image signals being indicative of locations and velocities of the reflectors; and
c) forming a 3-dimensional image 3-dimensionally indicating reflector velocities based on the image signals.

9. The method of Claim 8, further comprising preparing a mapping table of mapping colors with the respective velocities.

10. The method of Claim 9, wherein the step c) includes:
setting a velocity from the plurality of reflector velocities in the target object; and
forming the 3-dimensional image constructed with a plurality of voxels, wherein each of the voxels is formed to indicate 3-dimensional location based on the location and the reference velocity of the reflector and indicated by a color corresponding to the reference velocity by referring to the mapping table.

11. The method of Claim 9, wherein the step c) includes:
setting a reference velocity from the plurality of reflector velocities and setting a color corresponding to the reference velocity by referring to the mapping table; and
forming the 3-dimensional image constructed with a plurality of voxels, wherein each of the voxels is formed to indicate 3-dimensional location based on the location of the reflector and the color corresponding to the reference velocity of the reflector and indicated by the color corresponding to the reference velocity.

12. The method of Claim 9, wherein the step c) includes forming the 3-dimensional image constructed with a plurality of voxels, wherein each of the voxels is formed to indicate 3-dimensional location based on the location of the reflector and a current reflector velocity and indicated by the color corresponding to the current velocity.

13. The method of Claim 9, wherein the step c) includes:
setting a color corresponding to each of the reflector velocities by referring to the mapping table; and
forming the 3-dimensional image constructed with a plurality of voxels, wherein each of the voxels is formed to indicate 3-dimensional location based on the location of the reflector and the color corresponding to the current velocity and indicated by the color corresponding to the current velocity.

14. The method of Claim 9, wherein the steps c) includes:
setting a reference velocity from the plurality of reflector velocities and setting a color corresponding to each of the reflector velocities by referring to the mapping table; and
forming a 3-dimensional image constructed with a plurality of bar graphs, wherein each of the bar graphs is formed to indicate 3-dimensional location based on the location of the reflectors and the color corresponding to the current velocity and indicated by the color corresponding to the reference velocity.

15. The method of Claim 8, further comprising:
receiving reference plane setup information for setting a reference plane on the 3-dimensional image from the user;
setting the reference plane on the 3-dimensional image based on the reference plane setup information; and
forming a reference plane image corresponding to the reference plane.
